# EUROPÄISCHE PATENTANMELDUNG

(11) **EP 0 566 979 A1**
(43) Veröffentlichungstag der Anmeldung: **27.10.1993**
(21) Anmeldenummer: 93106092.5
(22) Anmeldetag: 15.04.1993
(51) Int. Cl.: A61K 7/06, A61K 7/48, A61K 31/57

(54) **Haarwasser und Haarbehandlungsverfahren zur Verringerung des Haarausfalls und Förderung des Haarwachstums bei androgenetischem Haarausfall**

(30) Priorität: 23.04.1992 DE 4213314
(71) Anmelder: Zingraf, Ingeborg, D-41464 Neuss (DE)
(72) Erfinder: Zingraf, Ingeborg, W-4040 Neuss (DE); Uphaus, Wolfgang, Dr. med., W-4040 Neuss (DE)
(74) Vertreter: Cramer, Eva-Maria

(57) **Zusammenfassung**

Gegenstand der Erfindung ist ein Mittel zur Behandlung der Haare und der Kopfhaut bei Haarausfall auf der Grundlage üblicher kosmetischer Träger- und Zusatzstoffe, wobei der haarwuchsfördernde Effekt auf der antiandrogenen Wirkung von Cyproteronacetat beruht. Diese Substanz ist geeignet, den androgenetischen Haarausfall - der bei Frauen auch bei nicht erhöhten Androgenwerten im Serum oder Sammelurin vorliegen kann - zu verringern und das Haarwachstum zu fördern.

Außerdem betrifft die Erfindung ein Haarbehandlungsverfahren zum gleichen Zweck, das sich auf die applizierte Menge des Haarwassers, die Häufigkeit der Anwendung und die Art und Weise der Anwendung bezieht.

Die perkutane Resorption des Cyproteronacetat gewährleistet eine Blockierung der Androgenrezeptoren der Kopfhaut, ohne daß bei der angegebenen Dosierung systemische Nebenwirkungen auftreten, die bei oraler oder parenteraler Applikation kaum zu vermeiden sind (z.B. Libidoverlust, Impotenz, reversible Zeugungsunfähigkeit, Gynäkomastie, thromboembolische Komplikationen, Leberfunktionsstörungen).

Zielgruppe für das Haarwasser und das Haarbehandlungsverfahren sind Männer und prä- und postklimakterische Frauen mit androgenetischer Teil- oder Hauptursache des Haarausfalls, die systemische Wirkungen des Cyproteronacetat vermeiden wollen.

## Beschreibung

Die Erfindung betrifft ein Mittel zur Behandlung des Haarausfalls und Förderung des Haarwachstums mit einem Gehalt an 6-chlor-17-hydroxy-1,2 -methylen-4,6-pregnadien-3,20-dionacetat (Cyproteronacetat).

Dieses Antiandrogen wird in oraler und parenteraler Applikationsform u.a. zur Behandlung der androgenetischen Alopezie eingesetzt. Diese Anwendungsform ist jedoch mit nicht unerheblichen Risiken, Nebenwirkungen und Gegenanzeigen belastet (thromboembolische Komplikationen, Libido- und Potenzminderung, reversible Zeugungsunfähigkeit, Gynäkomastie, depressive Verstimmungen, Leberfunktionsstörungen).

Es bestand daher die Aufgabe,ein Ersatzmittel für Frauen mit androgenetischem Haarausfall zu finden, bei denen aus medizinischen Gründen eine orale Therapie mit Antiandrogenen (z.B. "Androcur" und "Diane" von der Firma Schering und "Gestamestrol" von der Firma Hermal vertrieben) kontraindiziert ist, das die Androgenrezeptoren in der Kopfhaut blockiert, ohne unerwünschte systemische Nebenwirkungen (z.B. Gynäkomastie oder Impotenz bei Männern, Zyklusanomalien oder thromoboembolische Komplikationen bei Frauen) hervorzurufen.

Als Trägerstoff werden Wasser, Äthanol, Rizinusöl und Benzylbenzoat gewählt, die eine gute Lösung und perkutane Resorption des Wirkstoffs Cyproteronacetat gewährleisten, die Wirksamkeit des Cyproteronacetat nicht nachteilig beeinflussen, die Kopfhaut nicht schädigen und keine wesentliche allergene Potenz besitzen.

Die Erfindung betrifft Lösungen mit einem Gehalt an Cyproteronacetat zwischen 0,01 und 1 Gewichtsprozent.

Bei peri- und postklimakterischem Haarausfall hat sich eine Lösung mit 0,1 Gewichtsprozent Cyproteronacetat bewährt:

| Zusammensetzung: | |
|---|---|
| Cyproteronacetat | 0,100 g |
| Rizinusöl | 0,354 g |
| Benzylbenzoat | 0,619 g |
| 96%-iger Äthanol bis auf | 100 g |

Mit dieser 0,1%-igen Cyproteronlösung konnten bei peri- und postklimakterischen Frauen mit androgenetischem Haarausfall der Haarausfall in wenigen Wochen deutlich verringert werden und eine Zunahme des Haarwachstums in den nächsten Monaten erzielt werden.

Wenn keine medizinische Kontraindikation besteht, empfiehlt es sich, bei postklimakterischen Frauen zusätzlich eine orale Substitution mit konjugierten östrogenen und einem Gestagen mit antiandrogener Wirkung (z.B. Medrogeston) durchzuführen; bezüglich des Haarausfalls ist jedoch auch die alleinige Anwendung des Haarwassers mit Cyproteronacetat gut wirksam.

Auch für präklimakterische Frauen mit androgenetischem Haarausfall, die Nebenwirkungen einer oralen Therapie mit Antiandrogenen nicht in Kauf nehmen wollen, stellt die Anwendung des Haarwassers mit Cyproteronacetat eine wirksame Alternative dar.

Ähnliches gilt für Männer mit androgenetischem Haarausfall, die Nebenwirkungen einer systemischen Therapie mit Antiandrogenen vermeiden wollen.

Die vorliegende Erfindung betrifft weiterhin ein Haarbehandlungsverfahren zur Verringerung des Haarausfalls und Förderung des Haarwachstums, welches dadurch gekennzeichnet ist, daß man eine ausreichende Menge des Haarwassers (bei 0,1 Gewichtsprozent Cyproteronacetat maximal 30 ml des Haarwassers pro Woche zur Vermeidung systemischer Wirkungen) mittels einer Tropfpipette auf die Kopfhaut (bzw. die betroffenen Stellen der Kopfhaut) aufbringt, anschließend die Kopfhaut eine Minute massiert und dann mit einer Plastikfolie für 30 Minuten luftdicht abdeckt. Die Folienabdeckung ist für eine gute perkutane Resorption und passagere Durchblutungssteigerung der Kopfhaut wichtig.

Das Mittel ist dann bis zur nächsten Applikation auf der Kopfhaut zu belassen. Nach jedem Waschen der Haare ist das Mittel erneut in der beschriebenen Weise zu applizieren.
Eine Kombination mit anderen Haarpflegemitteln, die entfettende Wirkung haben, sollte vermieden werden. Der Gebrauch von Haarspray ist ohne Wirksamkeitsnachlaß des Haarwassers möglich.

Eine Behandlung mit dem Haarwasser bei androgenetischem Haarausfall führt in vielen Fällen nach einer Zeit von 3-6 Monaten zu einer deutlichen Verringerung des Haarausfalls und Zunahme des Haarwachstums. Die Anwendung ist als Langzeittherapie angelegt.

### Empfohlene Konzentrationen von Cyproteronacetat im Haarwasser:

Das Haarwasser sollte in drei Konzentrationsstufen auf dem Markt angeboten werden, um den individuellen Gegebenheiten der Anwender Rechnung zu tragen und das Risiko evtl. systemischer Nebenwirkungen zu minimieren.

Der individuelle Bedarf des Anwenders wird vom Hormonstatus und Androgenrezeptorstatus der Kopfhaut abhängen.
Aussagekräftige laborchemische Testmethoden zu diesen individuellen Voraussetzungen liegen nicht vor, da selbst im Normbereich liegende Hormonkonzentrationen einen androgenetischen Haarausfall nicht ausschließen können.

In der praktischen Anwendung wird man sich deshalb nach rein empirischen Kriterien der individuell erforderlichen Konzentration und Dosierung nähern müssen.
Es empfiehlt sich daher, zunächst von einer mittleren Konzentration und Dosierung auszugehen und abhängig vom Therapieverlauf diese zu steigern oder zu vermindern, bis die individuelle Erhaltungsdosis gefunden ist.
Diese Dosis ist natürlich bei Änderung der hormonellen Grundsituation (z.B. beginnendes Klimakterium) entsprechend dem individuellen Bedarf zu modifizieren.

| **Zusammensetzung der verschiedenen Konzentrationen:** | | |
|---|---|---|
| 1. | Cyproteronacetat | 0,025 g |
| | Rizinusöl | 0,089 g |
| | Benzylbenzoat | 0,155 g |
| | 96%-iger Äthanol bis auf | 100 g |
| 2. | Cyproteronacetat | 0,050 g |
| | Rizinusöl | 0,177 g |
| | Benzylbenzoat | 0,310 g |
| | 96%-iger Äthanol bis auf | 100 g |
| 3. | Cyproteronacetat | 0,100 g |
| | Rizinusöl | 0,354 g |
| | Benzylbenzoat | 0,619 g |
| | 96%-iger Äthanol bis auf | 100 g |

### Zielgruppen für die Anwendung des Haarwassers:

Positive therapeutische Wirkungen durch die Anwendung des cyproteronhaltigen Haarwassers sind bei allen Formen der Alopezie zu erzielen, die eine androgenetische Teilursache aufweisen.

Als Zielgruppen bieten sich insbesondere an, Männer mit typischer Alopezie (z.B. Geheimratsecken und Hinterhauptsglatze), Frauen im Präklimakterium, Klimakterium und Postklimakterium, sowie Frauen mit einem Climacterium praecox (z.B. Zustand nach diversen Intoxikationen mit Störungen der peripheren Gonadenfunktion und/oder der übergeordneten Regulationssysteme).

Vor Beginn der Behandlung sollten auf jeden Fall eine gynäkologische Untersuchung und ein Hormonstatus durchgeführt werden. Außerdem sollten evtl. sonstige Ursachen des Haarausfalls (z.B. Intoxikationen und Schilddrüsenerkrankungen) ausgeschlossen werden. Außerdem empfiehlt es sich, durch Vitaminstatus, Blut- und Haarmineralanalyse evtl. Mangelzustände abzuklären.

### Formen der Anwendung:

Ist die Behandlung der gesamten Kopfhaut erforderlich, so empfiehlt es sich, eine Applikation des Haarwassers zweimal pro Woche nach der Haarwäsche durchzuführen und das Haarwasser bis zur nächsten Haarwäsche auf der Kopfhaut zu belassen.

Bei Behandlungsbedürftigkeit der gesamten Kopfhaut sollte zur Vermeidung von systemischen Nebenwirkungen eine Gesamtdosis von 30 ml des 0,1%-igen Haarwassers pro Woche nicht überschritten werden.
Als Anfangsdosis empfiehlt sich 20 - 30 ml eines 0,05%-igen Haarwassers zu verwenden.

Sollte eine tägliche Haarwäsche erforderlich oder gewünscht sein, empfiehlt sich die Verwendung des 0,025%-igen Haarwassers in einer Maximaldosierung bis 120 ml pro Woche.

Ist der Haarausfall auf umschriebene Bereiche der Kopfhaut begrenzt (z.B. Geheimratsecken, Stirnglatze oder Hinterhauptsglatze), wie es gerade beim androgenetischen Haarausfall recht häufig vorkommt, ist es ausreichend, lediglich diese Bezirke zu behandeln.

Abhängig von den Haarwaschgewohnheiten sind bezüglich Konzentration und Dosierung die obigen Ausführungen zu beachten, insgesamt wird man jedoch abhängig von der Größe der betroffenen Bezirke entsprechend geringere Mengen des Haarwassers auftragen müssen.

Bei Frauen im Präklimakterium ist unabhängig davon, ob Ovulationshemmer oder sonstige Hormonpräparate zusätzlich genommen werden, und bei Frauen im Postklimakterium, die oral mit einem östrogen-Gestagen-Präparat substituiert werden, eine auf den Zyklus abgestimmte Applikation des Haarwassers zur Vermeidung von Blutungsanomalien zu empfehlen.
1. Beispiel:
   Einnahme eines Kombinationspräparates einer Frau im Postklimakterium (z.B. Presomen compositum).
   In diesem Fall sollte die Applikation des Haarwassers letztmalig am 21. Zyklustag erfolgen und am 4.Zyklustag des folgenden Zyklus wieder einsetzen.
   Diese Form der Anwendung sollte ebenfalls angewendet werden von Frauen im Präklimakterium, die Ovulationshemmer einnehmen.
2. Beispiel:
   Bei Frauen im Postklimakterium, die keine Hormonpräparate nehmen oder hormonell nicht-zyklisch behandelt werden, kann das Haarwasser im Bedarfsfall bis zur oben angegebenen Höchstdosierung (0,1%-ig 30 ml - 0,05%-ig 60 ml - 0,025%-ig 120 ml) kontinuierlich angewendet werden.
   Diese kontinuierliche Anwendung gilt ebenso für Männer.

Mittels einer Tropfpipette wird die vorgegebene Menge Haarwasser auf die gesamte Kopfhaut (bzw. auf die betroffenen Bereiche wie z.B. seitlicher Haaransatz und Hinterkopf) aufgetragen. Anschließend wird die Kopfhaut für ca. 1 Minute massiert.

Bei längeren Haaren empfiehlt es sich, das Haar vor der Anwendung hoch zu kämmen und mit einer Spange zu befestigen. Erst dann wird mit der Tropfpipette die vorgegebene Menge Haarwasser direkt auf die Kopfhaut aufgetragen und einmassiert.
Das aufgesteckte Haar verhindert ein Ablaufen des Haarwassers von der Kopfhaut zu den Haarspitzen.

Nach dem Auftragen des Haarwassers und dem Einmassieren wird die Kopfhaut mit einer Plastikfolie für ca. 30 Minuten luftdicht abgedeckt.
Bei täglicher Anwendung des Haarwassers auf einzelne Bereiche, wie z.B. Haaransatz und/oder Hinterkopf ist die Folienabdeckung nicht unbedingt erforderlich.
über die Plastikfolie wird ein Tuch fest um den Kopf gebunden, um das aufgetragene Haarwasser fest an die Kopfhaut zu drücken und um den Kopf warm zu halten, was sich auch günstig auf die Durchblutung und damit auch auf die perkutane Resorption des Haarwassers bis in die Haarpapille auswirkt.
Danach können wie gewohnt andere Haarpflegeprodukte verwendet werden, wenn sie nicht entfettend auf die Kopfhaut wirken.

### Kasuistik I:

Eine 53-jährige Frau mit postklimakterisch aufgetretenem Haarausfall im Bereich des Haaransatzes und der vorderen Scheitelregion applizierte 3-mal wöchentlich jeweils 2 ml des 0,1%-igen Haarwassers auf die betroffenen Stellen der Kopfhaut.

Der Haarausfall stoppte innerhalb von 2 Monaten, der Haaransatz wurde deutlich dichter und 4 Monate nach Behandlungsbeginn war der präklimakterische Haarstatus widerhergestellt. Unerwünschte Nebenwirkungen wurden nicht beobachtet.

Nach befriedigendem Behandlungsergebnis wurde die Behandlung mit dem Haarwasser für 6 Monate ausgesetzt. Nach dieser therapiefreien Zeit trat erneut verstärkter Haarausfall an den vorgenannten Regionen wieder auf.

Es wäre deshalb anzuraten, Therapiepausen für maximal 3 Monate einzulegen.

### Kasuistik II:

Eine 46-jährige Probandin mit Climacterium praecox im 35. Lebensjahr durch Ovarialinsuffizienz nach Chemikalienintoxikation litt seit der Intoxikation an Haarausfall unterschiedlicher Intensität.
Der Haarausfall betraf den gesamten behaarten Kopf mit Bevorzugung des Hinterkopfes und der Schläfenregion.

Ein Therapieversuch mit dem cyproteronhaltigen Ovulationshemmer Diane (Fa. Schering) zeigte nur eine geringe und unbefriedigende Wirkung.
Auch östrogenhaltige Haarwässer (Crinohermal der Fa. Hermal und Progynon B oleosum der Fa. Schering in alkoholischer Lösung) konnten den Haarausfall nicht positiv beeinflussen, hatten aber systemische Nebenwirkungen auf die Uterusschleimhaut.

Erst durch den Einsatz eines anderen Ovulationshemmers mit antiandrogener Wirkung (Gestamestrol der Fa. Hermal) konnte eine Verringerung des Haarausfalls erreicht werden.
Auch unter dieser Therapie wurde die alte Dichte und Länge des Haares nicht wieder erreicht; es bestand jedoch ein unter kosmetischem Aspekt akzeptabler Zustand.
Das Präparat mußte dann nach einer Beinvenenthrombose abgesetzt werden.

Trotz einer Hormonsubstitution mit konjugierten östrogenen und einem Gestagen mit leichter antiandrogener Wirkung (Presomen compositum) trat der Haarausfall wieder in verstärktem Ausmaß auf.
Auch eine zusätzliche lokale Behandlung der Kopfhaut mit Progynon B oleosum in alkoholischer Lösung konnte den Haarausfall nicht beeinflussen.

Erst durch die Anwendung des vorgenannten Haarwassers mit Cyproteronacetat und unter der Anwendung des Okklusivverbandes bei einer Anwendung von 30 ml Haarwasser pro Woche ließ sich der Haarausfall signifikant verringern und schüttere Stellen an den Schläfenregionen konnten durch vermehrtes Haarwachstum weitgehend beseitigt werden. Eine Anwendung ohne Okklusivverband brachte keinen Erfolg.

Eine Erhöhung der Dosierung war mit Blutungsanomalien verbunden und eine Verringerung führte wieder zu verstärktem Haarausfall.

Über einen Zeitraum von S Monaten wurde das Haarwasser abgesetzt, wobei es dann wieder zu einem verstärkten Haarausfall des gesamten Haupthaares kam.

Es ist bei dieser Probandin davon auszugehen, daß es sich um eine Dauerbehandlung auf Lebenszeit handeln wird.

### Kasuistik III:

Bei einem 38-jährigen Mann mit androgenetischem Haarausfall seit ca. 20 Jahren und seit ca. 15 Jahren bestehender Teilglatze im Stirn-, Scheitel- und Hinterkopfbereich führte die Applikation von 1 ml des o,1%-igen Haarwassers täglich über einen Zeitraum von 6 Monaten zu einem dichteren Haarwuchs im Bereich des auch schon schütteren Haarkranzes und zu einer neuen Haarbildung im Bereich der vormals haarlosen Kopfhautbereiche.

Dieser Effekt hält auch nach einer weiteren täglichen Applikation von 2 ml eines 0,02%-igen Haarwassers über 6 Monate an.

### Sonstige Erfahrungen mit dem cyproteronhaltigen Haarwasser:

Äthanol mit einer Konzentration unter 96% ist als Trägerstoff ungeeignet, da eine nur unvollständige Lösung des Cyproteronacetat resultiert und eine gleichmäßige und ausreichende perkutane Resorption durch niedrigere Konzentrationen dieses Trägerstoffs nicht gewährleistet ist.

Isopropylalkohol als Trägerstoff ist ebenfalls nicht zu empfehlen, da dieser nach Resorption zu Aceton metabolisiert werden kann und dieser Metabolit zu zentralnervösen Symptomen (Schwindel, Benommenheit, Kopfschmerzen) führen kann.

Auf weitere Zusatzstoffe (Farbstoffe, Konservierungsmittel, Parfumöle und sonstige Duftstoffe) sollte verzichtet werden, um bei Langzeitanwendung Allergisierungen zu vermeiden.

Nach Anwendung des Haarwassers sollten weitere Haarpflegeprodukte, die entfettenden Charakter haben, nicht zur Anwendung kommen, da es sonst zu einer Wirkungsabschwächung des Haarwassers, einer Austrocknung der Kopfhaut und zu einer Sprödigkeit der Haare kommen kann.

Das Haarwasser sollte bei Männern und Frauen nur nach ärztlicher Verordnung zur Anwendung kommen. Bei Frauen sollten im Verlauf der Behandlung regelmäßige gynäkologische Kontrolluntersuchungen alle 6 Monate erfolgen.

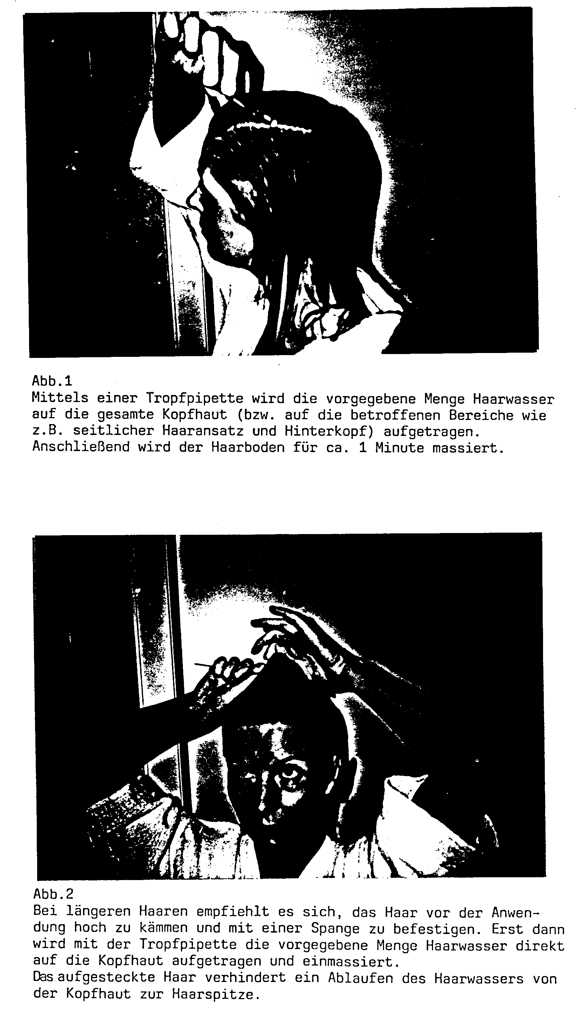

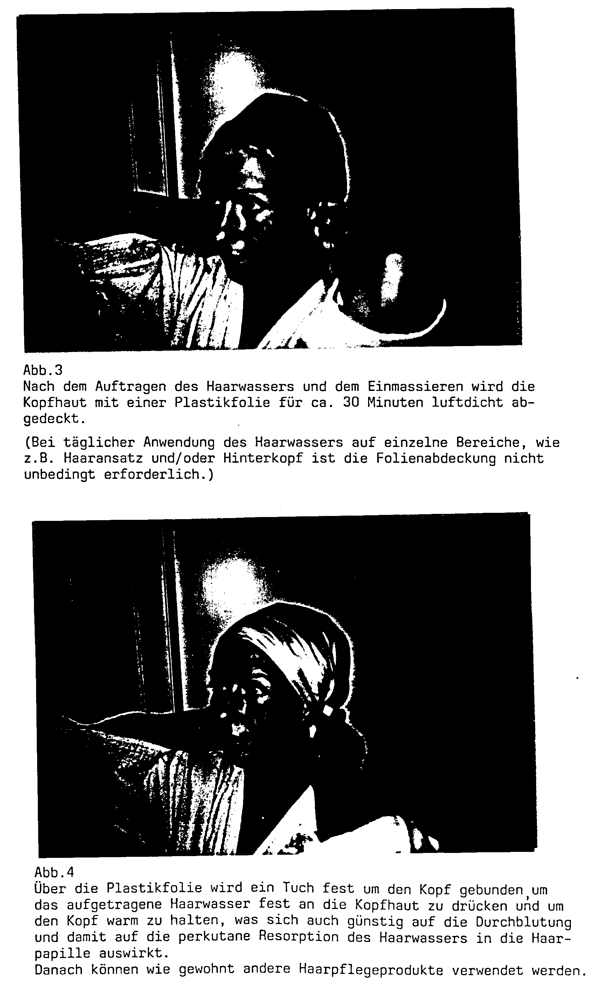

## Patentansprüche

1. Den Haarausfall verringerndes und das Haarwachstum förderndes, auf die Kopfhaut aufzutragendes Mittel, auf der Basis eines Aktivkomplexes, bestehend aus einer Kombination von Wirkstoffkomponenten und Träger- und Zusatzstoffen, wie Wasser, Äthanol, Rizinusöl und Benzylbenzoat oder Mischungen dieser Verbindungen,
dadurch gekennzeichnet, daß
- es als Wirkstoffkomponente Cyproteronacetat in alkoholischer Lösung enthält.

2. Mittel nach Anspruch 1, dadurch gekennzeichnet, daß der Gehalt an Cyproteronacetat 0,01 bis 1 Gewichtsprozent beträgt.

3. Verwendung von Cyproteronacetat in alkoholischer Lösung zur lokalen Behandlung von Haarausfall und Förderung des Haarwachstums bei allen Formen des Haarausfalls mit androgenetischer Teilursache - insbesondere bei Frauen im Klimakterium und Postklimakterium.

4. Verwendung nach Anspruch 3, dadurch gekennzeichnet, daß man eine ausreichende Menge des Haarwassers - mindestens 2 Anwendungen pro Woche - auf die Kopfhaut aufträgt, einmassiert, für 30 Minuten mittels eines Okklusivverbandes luftdicht abschließt und das Mittel anschließend mindestens bis zur nächsten Applikation auf der Kopfhaut beläßt.
